# EUROPEAN PATENT SPECIFICATION

(11) **EP 3 081 224 B1**
(45) Date of publication and mention of the grant of the patent: **05.06.2019**
(21) Application number: 15001119.5
(22) Date of filing: 16.04.2015
(51) Int. Cl.: A61K 36/899, A61K 36/31, A61P 7/06, A61P 25/00, A61P 25/02

(54) **ELYMUS AS A VITAMIN B12 SOURCE**
ELYMUS ALS QUELLE VON VITAMIN B12
ELYMUS EN TANT QUE SOURCE DE VITAMINE B12

(43) Date of publication of application: 19.10.2016
(73) Proprietor: Pandalis, Georgios, 49219 Glandorf (DE)
(72) Inventor: Pandalis, Georgios, 49219 Glandorf (DE)
(74) Representative: Grünecker Patent- und Rechtsanwälte PartG mbB

(56) References cited:
- EP-A2- 0 209 724
- WO-A2-2004/037165
- DE-A1- 2 922 467
- DATABASE WPI Week 201409 27 November 2013 (2013-11-27) Thomson Scientific, London, GB; AN 2014-B67014 XP002744907, & CN 103 404 720 A (JIN W) 27 November 2013 (2013-11-27)
- WATANABE F, YABUTA Y, TANIOKA Y, BITO T: "Biologically active vitamin B12 compounds in foods for preventing deficiency among vegetarians and elderly subjects", J AGRIC FOOD CHEM., vol. 61, 19 June 2013 (2013-06-19), pages 6769-6775, XP002744908, DOI: 10.1021/jf401545z

## Description

The present invention relates to at least one plant of the genus *Elymus* for use in the prophylaxis or treatment of a vitamin B₁₂ deficiency and to the non-therapeutical use of at least one plant of the genus *Elymus* as a vitamin B₁₂ source.

Vitamin B₁₂ is the collective name for cobalt-containing corrinoids that have biological activity in humans. The main cobalamins in humans and animals are hydroxocobalamin, adenosylcobalamin and methylcobalamin, the last two being the active coenzyme forms. Cyanocobalamin is a form of vitamin B₁₂ that is widely used clinically and for food supplementation due to its availability and stability. Vitamin B₁₂ is a water-soluble vitamin that is most stable at pH 4.0 to 4.5. It is stable to heat between pH 4 and 7 but should be protected from extremes of pH, strong light and oxidizing agents.

Vitamin B₁₂ plays a key role in the normal functioning of the brain and nervous system, and for the formation of blood. It is one of the eight B vitamins. It is normally involved in the metabolism of every cell of the human body, especially affecting DNA synthesis and regulation, but also fatty acid metabolism and amino acid metabolism (Yamada, Kazuhiro (2013) "Chapter 9. Cobalt: Its Role in Health and Disease" in Astrid Sigel, Helmut Sigel and Roland K. O. Sigel: Interrelations between Essential Metal Ions and Human Diseases. Metal Ions in Life Sciences 13; Springer, pp. 295-320). Fungi, plants, or animals are not capable of producing vitamin B₁₂. Only microorganisms such as bacteria and archaea have the enzymes required for its synthesis. These microorganisms also exist as symbionts in the intestinal tract of animals and on the surface of herbal hosts (Claus Leitzmann et al.: 21. Vitamin B12 (Cobalamin), Ernährung in Prävention und Therapie: ein Lehrbuch, 2nd edition, Georg Thieme Verlag 2003; p. 51).

Vitamin B₁₂ deficiency or hypocobalaminemia typically features a low blood level of vitamin B₁₂. However, functional B₁₂ deficiency can occur at any serum level with or without anaemia and/or macrocytosis. The deficiency is common to all age groups and is sometimes diagnosed late due to the lack of a gold standard assay and its complex aetiology. Neuropsychiatric symptoms can precede hematologic signs and are often the presenting manifestation of B₁₂ deficiency. Vitamin B₁₂ deficiency can cause permanent damage to nervous tissue if left untreated longer than 6 months (Lindenbaum J, Healton E, Savage D, Brust J, Garrett T, Podell E et al., Jun 1988, Neuropsychiatric disorders caused by cobalamin deficiency in the absence of anemia or macrocytosis; The New England Journal of Medicine 318 (26): pp. 1720-8).

One reason for a vitamin B₁₂ deficiency is an inadequate dietary intake of vitamin B₁₂. Vitamin B₁₂ occurs in animal products (eggs, meat, milk) and recent research indicates it may also occur in some algae, such as *Chlorella* and *Susabi-nori* (*Porphyra yezoensis*). Vegans and also vegetarians may be at risk for vitamin B₁₂ deficiency due to inadequate dietary intake of vitamin B₁₂, if they do not supplement. However, vitamin B₁₂ deficiency can occur even in people who consume meat, poultry, and fish (McBride, Judy, 2 August 2000, B12 Deficiency May Be More Widespread Than Thought, Agricultural Research Service; United States Department of Agriculture).

3 µg of vitamin B₁₂ are recommended as a daily dose for adults by the German Nutrition Society.

*Elymus* is a genus of perennial plants in the grass family, related to rye, wheat, and other widely grown cereal grains, which belongs to the family of *Poaceae* (Linnaeus, Carl von; 1753; Species Plantarum 1: pp. 83-84). *Elymus* is widespread across all parts of the northern hemisphere having a moderate climate and the south of South America. The term *Elymus* is synonymous with the term *Agropyron. Elymus repens (Agropyron repens),* commonly known as couch grass, is native to most of Europe, Asia, the Arctic biome and northwest Africa. *E*. *repens* is known to be a medicinal plant. The dried rhizomes of *E*. *repens* were broken up and used as incense in mediaeval Northern Europe. *E. repens* rhizomes have also been used in traditional medicine against fever, internally as a tea, syrup, or cold maceration in water, or externally applied as a crude drug. In addition, they were traditionally used as a diuretic in urinary tract infections or for the prevention of renal gravel (Bisset NG, ed. Herbal Drugs and Phytopharmaceuticals; 2nd ed. Stuttgart, Germany: Medpharm Scientific Publishers; 2001).

The *Fabaceae* or *Leguminosae*, commonly known as the legume, pea, or bean family, are a large and economically important family of flowering plants. It includes trees, shrubs, and herbaceous plants perennials or annuals, which are easily recognized by their fruit (legume) and their compound, stipulated leaves. The group is widely distributed and is the third-largest land plant family in terms of number of species, behind only the *Orchidaceae* and *Asteraceae*, with 630 genera and over 18,860 species (Judd, W. S., Campbell, C. S. Kellogg, E. A. Stevens, P.F. Donoghue, M. J. (2002), Plant systematics: a phylogenetic approach, Sinauer Axxoc, pp. 287-292). The five largest of the 630 legume genera are *Astragalus* (over 2,000 species), *Acacia* (over 1000 species), *Indigofera* (around 700 species), *Crotalaria* (around 700 species), and *Mimosa* (around 500 species), which constitute about a quarter of all legume species. About 18,000 legume species are known, amounting to about 7% of flowering plant species. *Fabaceae* is the most common family found in tropical rainforests and in dry forests in the Americas and Africa (Burnham, R. J., & Johnson, K. R. 2004. South American palaeobotany and the origins of neotropical rain forests. Phil. Trans. Roy. Soc. London B, 359: pp. 1595-1610).

The *Poaceae* (also called *Gramineae* or true grasses) are a large and nearly ubiquitous family of monocotyledonous flowering plants. With more than 10,000 domesticated and wild species, the *Poaceae* are the fifth-largest plant family, following the *Asteraceae*, *Orchidaceae*, *Fabaceae*, and *Rubiaceae.*

The *Brassicaceae,* a medium-sized and economically important family of flowering plants (Angiosperms), are informally known as the mustards, mustard flowers, the crucifers or the cabbage family. The name *Brassicaceae* is derived from the included genus *Brassica. Cruciferae*, an older name, meaning "cross-bearing", describes the four petals of mustard flowers, which are reminiscent of a cross; it is one of eight plant family names without the suffix '-aceae' that are authorized alternative names (according to ICBN Art. 18.5 and 18.6 Vienna Code), thus both *Cruciferae* and *Brassicaceae* are used. The family contains over 330 genera and about 3,700 species, according to the Royal Botanic Gardens, Kew.

*Elaeagnaceae,* the oleaster family, is a plant family of the order Rosales comprising small trees and shrubs, native to temperate regions of the Northern Hemisphere, south into tropical Asia and Australia. The family has 45-50 species in three genera. They are commonly thorny, with simple leaves often coated with tiny scales or hairs. Most of the species are xerophytes (found in dry habitats); several are also halophytes, tolerating high levels of soil salinity.

WO-A-96/19490 discloses vitamin B₁₂-containing sallow thorn concentrates or extracts.

The object of the present invention is to provide a plant-based source of vitamin B₁₂ which is suitable for the prophylaxis or treatment of a vitamin B₁₂ deficiency or for a non-therapeutical use as a vitamin B₁₂ source.

This object is achieved by at least one plant of the genus *Elymus* for therapeutical use in the prophylaxis or treatment of a vitamin B₁₂ deficiency.

Another embodiment of the present invention is directed to the non-therapeutical use of at least one plant of the genus *Elymus* as a vitamin B₁₂ source.

Preferred embodiments are set forth in subclaims 2, 3, and 5 to 10.

In a preferred embodiment in combination with any of the above or below embodiments, the at least one plant of the genus Elymus is native to Europe, more preferably selected from the group consisting of *E. repens*, *E. cristatum, E. caninus*, E. junceus, *E. biflorum*, and *E*. *intermedium,* in particular *E. repens.*

All parts of the at least one plant of the genus *Elymus* can be used for the purpose of the present invention. Preferably, the root of the plant is used.

In a preferred embodiment in combination with any of the above or below embodiments, at least one plant of the genus *Elymus* is used in combination with one or more additional plants selected from the families of *Fabaceae*, *Poaceae*, *Brassicaceae* and *Elaeagnaceae.*

In a preferred embodiment in combination with any of the above or below embodiments, at least one plant of the genus *Elymus* is used in combination with one or more additional plants selected from the family of *Fabaceae.* In another preferred embodiment in combination with any of the above or below embodiments, the one or more additional plants from the family of *Fabaceae* are selected from *Trigonella foenum graecum* and *Cicer arietinum.*

In a preferred embodiment in combination with any of the above or below embodiments, at least one plant of the genus *Elymus* is used in combination with one or more additional plants selected from the family of *Poaceae.* In another preferred embodiment in combination with any of the above or below embodiments, the one or more additional plants from the family of *Poaceae* are selected from *Echinochloa crus-galli* and *Zea mays.*

In a preferred embodiment in combination with any of the above or below embodiments, at least one plant of the genus *Elymus* is used in combination with one or more additional plants selected from the family of *Brassicaceae.* In another preferred embodiment in combination with any of the above or below embodiments, the one or more additional plants from the family of *Brassicaceae* are selected from *Alliaria officinalis* and *Capsella bursa-pastoris.* In a another preferred embodiment in combination with any of the above or below embodiments, the additional plant from the family of *Brassicaceae* is mustard, more preferably black mustard and, in particular, black mustard seeds.

In a preferred embodiment in combination with any of the above or below embodiments, at least one plant of the genus *Elymus* is used in combination with one or more additional plants selected from the family of *Elaeagnaceae.* In another preferred embodiment in combination with any of the above or below embodiments, the additional plant from the family of *Elaeagnaceae* is sallow thorn.

All parts of the additional plant can be used for the purpose of the present invention. Preferably, the root or seed of the plant is used.

In a preferred embodiment, an extract or concentrate of at least one plant of the genus *Elymus* is used. The extract or concentrate can be obtained in a manner known per se.

In another preferred embodiment, an extract or concentrate of at least one plant of the genus *Elymus* and one or more additional plants selected from the families of *Fabaceae*, *Poaceae, Brassicaceae* and *Elaeagnaceae* is used. The extract or concentrate can be obtained in a manner known per se.

According to the present invention, the term "extract" is used representatively for all products that are obtained from an herbal subject by means of an extraction with a solvent, such as with maceration or percolation. The term "concentrate" is used representatively for all products that are obtained from an herbal subject by means of removal of water from the fresh plant. A dry plant product, such as a powder or a comminuted plant, is also included in the term "concentrate".

Generally, an extraction of the plant parts including roots, seeds, leaves, twigs, blossoms, buds and fruits with a suitable solvent takes place. A suitable solvent can be selected depending on what ingredients are to be extracted apart from vitamin B₁₂. Suitable solvents are water, alcohols, such as methanol, ethanol or isopropyl alcohol, aliphatic hydrocarbons, such as hexane, or chlorinated solvents, such as dichloromethane, as well as acetone, acetylacetone, ethylacetate, ammonia or glacial acetic acid, but also supercritical carbon dioxide. Mixtures of the solvents mentioned can also be used. In a preferred embodiment, in combination with any one of the embodiments listed above or below, water or a mixture of water with methanol or ethanol is used. In another preferred embodiment, an aqueous solution having a pH is 4.0 to 4.5 is used.

The extraction is normally carried out at room temperature, e.g. at about 18 to 25°C, or at a temperature of 25°C to, where applicable, as high as the boiling point of the solvent used. Preferred is an extraction at 95 to 100°C.

The extraction is normally carried out for 2 to 8 h. Preferably, the extraction is carried out for 3 to 6 h, more preferably for 4 to 5 h. Furthermore, fats, such as pork fat, waxes, such as beeswax, or oils, such as olive oil and almond oil, can be used for the extraction. Preferably, almond oil is used.

In order to achieve the highest possible yield, the plant material can be extracted a number of times. Preferably, the extraction is repeated 2 to 6 times, more preferably 3 times. In this case, it is also possible to use different solvents in the various extraction steps or an extraction with a solvent can be followed by an extraction with a fat, wax or oil, or vice versa.

As a result of the extraction, a liquid, semi-solid or solid raw product is obtained, which can be used in this form for the purposes of the present invention.

A maceration procedure is normally performed for five to nine days, preferably for seven days, at room temperature with a mixture of water and ethanol, by pouring the solvent mixture over the plant elements and letting this stand for the period of time mentioned.

According to the present invention, a percolation of the plant parts is normally achieved by treating the parts with water at 95 to 100°C for four to five hours by conducting the water through the plant parts.

The crude product obtained from an extraction with a solvent, such as a maceration or percolation, can also be concentrated and/or dried and/or further processed before use. The further processing can, for example, include cleaning steps known to the person skilled in the art, such as centrifugation, filtration and decanting, in order to remove suspended materials from the extract. Chromatography, such as column chromatography, gas chromatography or HPLC or steam distillation may also be used for purification. In a preferred embodiment the crude product is used without further purification steps.

An extract obtained in this way can subsequently be further processed into a dry extract. To produce the dry extract, the solvent can be withdrawn from the liquid raw extract, the concentrated extract or the cleaned extract by, for example, spray drying, freeze drying or vacuum drying.

In a further preferred embodiment in combination with one of the embodiments listed above or below, the composition according to the present invention comprises a concentrate or extract of at least 2, 3, or 4 plants selected from at least one plant of the genus *Elymus* and one or more additional plants selected from the families of *Fabaceae*, *Poaceae*, *Brassicaceae* and *Elaeagnaceae.* More preferably, the composition comprises a concentrate or extract of two or three plants.

For preparing the concentrate or extract used according to the present invention, preferably the roots, seeds, leaves, twigs, blossoms and buds, more preferably the roots or seeds, are used.

In the present invention, the term "twig" refers to a small shoot or branch having a diameter of 3 cm at most. Preferably, the diameter of the twigs is up to 1 cm.

In an alternative preferred embodiment in combination with one of the embodiments listed above or below, the concentrate or extract is of at least one plant of the genus *Elymus*, more preferably the roots of the at least one plant of the genus *Elymus*, in particular *E. repens*, and one or more plants selected from the family of *Fabaceae.*

In an alternative preferred embodiment in combination with one of the embodiments listed above or below, the concentrate or extract is of at least one plant of the genus *Elymus,* more preferably the roots of the at least one plant of the genus *Elymus,* in particular *E. repens,* and one or more plants selected from the family of *Poaceae.*

In an alternative preferred embodiment in combination with one of the embodiments listed above or below, the concentrate or extract is of at least one plant of the genus *Elymus,* more preferably the roots of the at least one plant of the genus *Elymus,* in particular *E. repens,* and one or more plants selected from the family of *Brassicaceae.*

In an alternative preferred embodiment in combination with one of the embodiments listed above or below, the concentrate or extract is of at least one plant of the genus *Elymus,* more preferably the roots of the at least one plant of the genus *Elymus*, in particular *E*. *repens*, and one or more plants selected from the family of *Elaeagnaceae.*

The content of vitamin B₁₂ can be determined by a competitive enzyme immunoassay. This assay is described in Deutsche Lebensmittelrundschau, 86th year, issue 10, 1990, p. 307-310.

Surprisingly, it was found that the roots of *E*. *repens* contain a high amount of vitamin B₁₂. Vitamin B₁₂ was found in concentrations of 23.1 and 28.5 µg/100 g in a dry extract of the root of *E. repens* and in ground roots of *E*. *repens*, respectively. In addition, vitamin B₁₂ was found in concentrations of 17.98 and 13.26 µg/100 g in a dry and a ground extract of black mustard seeds, respectively.

The concentration of vitamin B₁₂ in the roots of *E*. *repens* is comparable to the vitamin B₁₂ levels of pig liver (25-39 µg/100 g), while other foods, such as milk with a level of about 0.4 µg/100 g and eggs with a level of about 2.5-3 µg/100 g, contain only a relatively small amount of vitamin B₁₂.

At least one plant, such as *E*. *repens,* of only the genus *Elymus* or in combination with one or more additional plants selected from the families of *Fabaceae, Poaceae, Brassicaceae* and *Elaeagnaceae* can thus be successfully used as a vitamin B₁₂ source, in particular, for the treatment or prophylaxis of a vitamin B₁₂ deficiency or in a non-therapeutical use as a vitamin B12 source in order to compensate for an inadequate dietary intake of vitamin B₁₂ as may occur, for example, in vegetarians or vegans.

In a preferred embodiment in combination with one of the embodiments listed above or below, the vitamin B₁₂ deficiency is a neuropathy and/or a disorder of erythropoiesis.

In an alternative preferred embodiment in combination with one of the embodiments listed above or below, the least one plant of the genus *Elymus* is for use in the stimulation of nucleic acid synthesis.

In the present invention, the term "prophylaxis" refers to a procedure to prevent a disease. Prophylactic measures can be divided into primary prophylaxis (prevention of the disease) and secondary prophylaxis (protection from the disease).

In another preferred embodiment in combination with one of the embodiments listed above or below, the extract or concentrate of the at least one plant of the genus *Elymus* is in liquid, dry or semi-solid form.

In another preferred embodiment in combination with one of the embodiments listed above or below, the extract or concentrate of the at least one plant of the genus *Elymus* and one or more additional plants selected from the families of *Fabaceae*, *Poaceae*, *Brassicaceae* and *Elaeagnaceae* is in liquid, dry or semi-solid form.

In a further preferred embodiment in combination with one of the embodiments listed above or below, the extract of the at least one plant of the genus *Elymus* is an aqueous extract, an alcoholic extract or an oily extract.

In a further preferred embodiment in combination with one of the embodiments listed above or below, the extract of the at least one plant of the genus *Elymus* and one or more additional plants selected from the families of *Fabaceae*, *Poaceae*, *Brassicaceae* and *Elaeagnaceae* is an aqueous extract, an alcoholic extract or an oily extract.

In a preferred embodiment in combination with one of the embodiments listed above or below, the extract or concentrate of the least one plant of the genus *Elymus* and optionally one or more additional plants selected from the families of *Fabaceae, Poaceae, Brassicaceae* and *Elaeagnaceae* is in the form of a tablet, capsule, powder, tea mixture, granulate, sirup, drops, tincture, emulsion, ointment, cream, gel, paste, infusion, inhalation solution or mouth spray.

The extract or concentrate of the at least one plant of the genus *Elymus* and optionally one or more additional plants selected from the families of *Fabaceae, Poaceae, Brassicaceae* and *Elaeagnaceae* (in the following designated as "composition") can be applied in each of the application forms familiar to the person skilled in the art for both medical and non-medical use.

In a preferred embodiment in combination with one of the embodiments listed above or below, the composition is applied in solid form as a tablet, such as a coated tablet, effervescent tablet, sugar-coated tablet, chewing tablet, lozenge, pill, chewing gum, capsule, powder, tea mixture, or granulate. In an alternative preferred embodiment in combination with one of the embodiments listed above or below, the composition is applied in liquid form as mouthwash, sirup, drops, tincture, emulsion, infusion, inhalation solution or gargling solution. In another preferred embodiment in combination with one of the embodiments listed above or below, the composition is applied in a semi-solid form as ointment, cream, gel, or paste. In a further preferred embodiment in combination with one of the embodiments listed above or below, the composition is applied as a spray, such as a mouth spray. In particular, the composition is applied as a tablet, sirup, tincture, tea mixture, and infusion.

In galenic and other application forms, the composition can be processed with customary galenic aids, such as tablet bonders, filling agents, preservative agents, tablet-opening agents, flow regulation agents, softening agents, wetting agents, dispersing agents, emulsifying agents, solvents, retarding agents, anti-oxidative agents, consistency regulators, penetration improvers and/or propellant gases. Further elements, such as vitamins and minerals, can be added to the composition according to the present invention.

The concentration of the composition varies, depending on the type of application. As a rule, the quantity is between 0.5 and 1,000 mg per dosing unit for solid application forms. Preferably, the quantity is between 1 and 500 mg per unit. In liquid application forms, the composition can be in a concentration of 0.1 µg/mL to 500 mg/ml, preferably from 1 µg/mL to 200 mg/mL, more preferably from 1 to 100 mg/mL, in particular 5 to 50 mg/mL. In the case of semi-solid application forms, the content of the composition amounts to 1 to 90% by weight, preferably 5 to 75 % by weight, based on the total weight of the application form.

### Examples

### General procedure for the production of liquid and dry extracts:

The collected plant material is visually checked, and non-original, damaged or eaten away parts are removed.

The purified material is spread on a table in a green house and covered with paper for drying. The plant parts are turned around on a daily basis and visually checked for non-original and damaged parts, which are removed. The residual moisture of the plant material is determined on regular basis. The material is good for further processing when the residual water content is at a maximum of 10% by weight. Alternatively, the purified material is dried with a belt dryer according to standard procedures known to the person skilled in the art.

The plant material is coarsely cut and transferred to a beaker. Cold, distilled water is added (10 to 30-times the quantity of the plant material). The resulting mixture is heated on a hotplate while being stirred until it starts boiling. Simmering is continued for 1 h.

The hot liquid is strained, and the residual plant material is squeezed out. The resulting aqueous extract is filled directly in a bottle.

For the production of the dry extract, the liquid extract is filled in metal bowls and concentrated at 80°C in a drying oven until the solvent has completely evaporated. The residue is scraped out of the metal bowl and weighed. Alternatively, the liquid extract is freeze-dried according to standard procedures known to the person skilled in the art.

### Roots of E. repens

Vitamin B₁₂ was found in concentrations of 23.1 and 28.5 µg/100 g in a dry extract of the roots of *E. repens* and in ground roots of *E. repens,* respectively. The content of vitamin B₁₂ was determined by a competitive enzyme immunoassay as well as immunoaffinity chromatography (IAC) in combination with reversed phase high performance/pressure liquid chromatography (RP-HPLC).

### Black mustard seeds

Vitamin B₁₂ was found in concentrations of 17.98 and 13.26 µg/100 g in a dry extract of black mustard seeds and ground black mustard seeds, respectively. The content of vitamin B₁₂ was determined by a competitive enzyme immunoassay as well as immunoaffinity chromatography (IAC) in combination with reversed phase high performance/pressure liquid chromatography (RP-HPLC).

## Claims

1. At least one plant of the genus *Elymus* for therapeutical use in the prophylaxis or treatment of a vitamin B₁₂ deficiency.

2. At least one plant of the genus *Elymus* for use according to claim 1, wherein the vitamin B₁₂ deficiency is a neuropathy and/or a disorder of erythropoiesis.

3. At least one plant of the genus *Elymus* for use according to claim 1, wherein the prophylaxis or treatment of a vitamin B₁₂ deficiency is the stimulation of nucleic acid synthesis.

4. Non-therapeutical use of at least one plant of the genus *Elymus* as a vitamin B₁₂ source.

5. At least one plant of the genus *Elymus* for therapeutical use or non-therapeutical use of at least one plant of the genus *Elymus* according to any of claims 1 to 4, wherein an extract or concentrate of the at least one plant of the genus *Elymus* is used.

6. At least one plant of the genus *Elymus* for therapeutical use or non-therapeutical use of at least one plant of the genus *Elymus* according to any of claims 1 to 5, wherein the root of the at least one plant of the genus *Elymus* is used.

7. At least one plant of the genus *Elymus* for therapeutical use or non-therapeutical use of at least one plant of the genus *Elymus* according to any of claims 1 to 6, wherein the plant is selected from the group consisting of *E. repens*, *E. cristatum,* E. caninus, E. junceus, *E*. *biflorum* and *E*. *intermedium.*

8. At least one plant of the genus *Elymus* for therapeutical use or non-therapeutical use of at least one plant of the genus *Elymus* according to any of claims 1 to 7, wherein the at least one plant of the genus Elymus is used in combination with one or more additional plants selected from the families of *Fabaceae, Poaceae, Brassicaceae* and *Elaeagnaceae.*

9. At least one plant of the genus *Elymus* for therapeutical use or non-therapeutical use of at least one plant of the genus *Elymus* according to claim 8, wherein an extract or concentrate of the one or more additional plants selected from the families of *Fabaceae*, *Poaceae*, *Brassicaceae* and *Elaeagnaceae* is used.

10. At least one plant of the genus *Elymus* for therapeutical use or non-therapeutical use of at least one plant of the genus *Elymus* according to claim 8 or 9, wherein the root of the one or more additional plants selected from the families of *Fabaceae, Poaceae, Brassicaceae* and *Elaeagnaceae* is used.

## Patentansprüche

1. Mindestens eine Pflanze der Gattung *Elymus* zur therapeutischen Verwendung bei der Prophylaxe oder Behandlung eines Vitamin-B₁₂-Mangels.

2. Mindestens eine Pflanze der Gattung *Elymus* zur Verwendung nach Anspruch 1, wobei der Vitamin-B₁₂-Mangel eine Neuropathie und/oder eine Störung der Erythropoese ist.

3. Mindestens eine Pflanze der Gattung *Elymus* zur Verwendung nach Anspruch 1, wobei die Prophylaxe oder Behandlung eines Vitamin-B₁₂-Mangels die Stimulierung der Nukleinsäuresynthese ist.

4. Nicht-therapeutische Verwendung von mindestens einer Pflanze der Gattung *Elymus* als Vitamin-B₁₂-Quelle.

5. Mindestens eine Pflanze der Gattung *Elymus* zur therapeutischen Verwendung oder nicht-therapeutische Verwendung von mindestens einer Pflanze der Gattung *Elymus* nach einem der Ansprüche 1 bis 4, wobei ein Extrakt oder Konzentrat der mindestens einen Pflanze der Gattung *Elymus* verwendet wird.

6. Mindestens eine Pflanze der Gattung *Elymus* zur therapeutischen Verwendung oder nicht-therapeutische Verwendung von mindestens einer Pflanze der Gattung *Elymus* nach einem der Ansprüche 1 bis 5, wobei die Wurzel der mindestens einen Pflanze der Gattung *Elymus* verwendet wird.

7. Mindestens eine Pflanze der Gattung *Elymus* zur therapeutischen Verwendung oder nicht-therapeutische Verwendung von mindestens einer Pflanze der Gattung *Elymus* nach einem der Ansprüche 1 bis 6, wobei die Pflanze ausgewählt ist aus der Gruppe, bestehend aus *E*. *repens*, *E. cristatum*, *E. cacinus*, *E. junceus*, *E. biflorum* und *E. intermedium.*

8. Mindestens eine Pflanze der Gattung *Elymus* zur therapeutischen Verwendung oder nicht-therapeutische Verwendung von mindestens einer Pflanze der Gattung *Elymus* nach einem der Ansprüche 1 bis 7, wobei die mindestens eine Pflanze der Gattung *Elymus* in Kombination mit einer oder mehreren zusätzlichen Pflanzen, ausgewählt aus den Familien *Fabaceae*, *Poaceae*, *Brassicaceae* und *Elaeagnaceae*, verwendet wird.

9. Mindestens eine Pflanze der Gattung *Elymus* zur therapeutischen Verwendung oder nicht-therapeutische Verwendung von mindestens einer Pflanze der Gattung *Elymus* nach Anspruch 8, wobei ein Extrakt oder Konzentrat der einen oder mehreren zusätzlichen Pflanzen, ausgewählt aus den Familien *Fabaceae*, *Poaceae*, *Brassicaceae* und *Elaeagnaceae*, verwendet wird.

10. Mindestens eine Pflanze der Gattung *Elymus* zur therapeutischen Verwendung oder nicht-therapeutische Verwendung von mindestens einer Pflanze der Gattung *Elymus* nach Anspruch 8 oder 9, wobei die Wurzel der einen oder mehreren zusätzlichen Pflanzen, ausgewählt aus den Familien der *Fabaceae*, *Poaceae*, *Brassicaceae* und *Elaeagnaceae*, verwendet wird.

## Revendications

1. Au moins une plante du genre *Elymus* pour l'utilisation thérapeutique dans la prophylaxie ou le traitement d'une carence en vitamine B₁₂.

2. Au moins une plante du genre *Elymus* pour l'utilisation selon la revendication 1, la carence en vitamine B₁₂ étant une neuropathie et/ou un trouble d'érythropoïèse.

3. Au moins une plante du genre *Elymus* pour l'utilisation selon la revendication 1, la prophylaxie ou le traitement d'une carence en vitamine B₁₂ étant la stimulation de la synthèse d'acide nucléique.

4. Utilisation non thérapeutique d'au moins une plante du genre *Elymus* comme source de vitamine B₁₂.

5. Au moins une plante du genre *Elymus* pour l'utilisation thérapeutique ou l'utilisation non thérapeutique d'au moins une plante du genre *Elymus* selon l'une quelconque des revendications 1 à 4, un extrait ou un concentré de ladite plante du genre *Elymus* étant utilisé.

6. Au moins une plante du genre *Elymus* pour l'utilisation thérapeutique ou l'utilisation non thérapeutique d'au moins une plante du genre *Elymus* selon l'une quelconque des revendications 1 à 5, la racine d'au moins une plante du genre *Elymus* étant utilisée.

7. Au moins une plante du genre *Elymus* pour l'utilisation thérapeutique ou l'utilisation non thérapeutique d'au moins une plante du genre *Elymus* selon l'une quelconque des revendications 1 à 6, la plante étant sélectionnée dans le groupe constitué d'*E*. *repens, E. cristatum, E caninus, E. junceus, E. biflorum* et *E*. *intermedium.*

8. Au moins une plante du genre *Elymus* pour l'utilisation thérapeutique ou l'utilisation non thérapeutique d'au moins une plante du genre *Elymus* selon l'une quelconque des revendications 1 à 7, ladite plante du genre *Elymus* étant utilisée en combinaison avec une ou plusieurs plantes supplémentaires sélectionnées parmi les familles des *Fabaceae, Poaceae, Brassicaceae* et *Elaeagnaceae.*

9. Au moins une plante du genre *Elymus* pour l'utilisation thérapeutique ou l'utilisation non thérapeutique d'au moins une plante du genre *Elymus* selon la revendication 8, un extrait ou un concentré desdites plantes supplémentaires sélectionnées parmi les familles des *Fabaceae, Poaceae, Brassicaceae* et *Elaeagnaceae* étant utilisé.

10. Au moins une plante du genre *Elymus* pour l'utilisation thérapeutique ou l'utilisation non thérapeutique d'au moins une plante du genre *Elymus* selon la revendication 8 ou 9, la racine desdites plantes supplémentaires sélectionnées parmi les familles des *Fabaceae, Poaceae, Brassicaceae* et *Elaeagnaceae* étant utilisée.
